(19) Europäisches Patentamt
European Patent Office
Office européen des brevets

(11) Veröffentlichungsnummer: **0 221 026**
**B1**

(12) # EUROPÄISCHE PATENTSCHRIFT

(45) Veröffentlichungstag der Patentschrift: **19.04.89**

(51) Int. Cl.⁴: **C07C 149/18**, C07C 147/02, D06M 13/28

(21) Anmeldenummer: **86810473.8**

(22) Anmeldetag: **22.10.86**

(54) **Perfluoralkyl substituierte Pyromellitsäureester.**

(30) Priorität: **28.10.85 US 791817**

(43) Veröffentlichungstag der Anmeldung: **06.05.87 Patentblatt 87/19**

(45) Bekanntmachung des Hinweises auf die Patenterteilung: **19.04.89 Patentblatt 89/16**

(84) Benannte Vertragsstaaten: **DE FR GB IT**

(56) Entgegenhaltungen:
**US-A- 4 209 610**
**US-A- 4 317 736**

(73) Patentinhaber: **CIBA-GEIGY AG, Klybeckstrasse 141, CH-4002 Basel(CH)**

(72) Erfinder: **Karydas, Athanasios, 574 92nd Street, Brooklyn New-York 11209(US)**
Erfinder: **Falk, Robert A., 35 Glenside Drive, New City New-York 10956(US)**

EP 0 221 026 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Perfluoralkylalkyl-(sulfo- oder -sulfonyl-)-alkylester von Pyromellitsäure, die teilweise mit 3-Chlor-1,2-propandiol verestert sind, und ihre Verwendung zur wasser- und ölabweisenden Ausrüstung von synthetischen Polyestern, z.B. Polyethylenterephthalaten, und synthetischen Polyamiden, z.B. Nylon 6 oder Nylon 6,6, besonders Fasern aus diesen synthetischen Materialien, und ihre Verwendung um diese Materialien widerstandsfähig gegen Schmutz zu machen.

Fluoralkylgruppen enthaltende Ester von Pyromellitsäure sind z.B. in den US-Patenten 4 209 610, 4 283 292 und 4 317 736 beschrieben. Diese Ester enthalten keine Sulfo- oder Sulfonylbrückengruppen.

Die Fluoralkylgruppen enthaltenden Pyromellitsäureester werden im allgemeinen bei erhöhten Temperaturen auf die Fasern aufgebracht und die behandelten Fasern danach bei hohen Temperaturen getempert. Die thermische Stabilität solcher Pyromellitsäureester ist daher von grosser Bedeutung.

Es wurde überraschend gefunden, dass die erfindungsgemässen Pyromellitsäureester, besonders solche mit Sulfongruppen, eine erhöhte thermische Stabilität aufweisen.

Ein Gegenstand der Erfindung sind Perfluoralkylalkylsulfo- oder -sulfonyl-alkylester der Pyromellitsäure, die teilweise mit 3-Chlor-1,2-propandiol verestert sind, der Formeln I oder II,

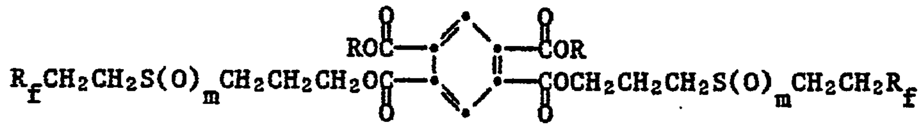 (I)

oder

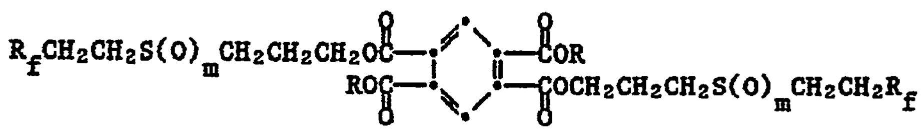 (II),

oder Mischungen davon,
worin
$R_f$ $C_3$–$C_{18}$-Perfluoralkyl, m 0 oder 2, und

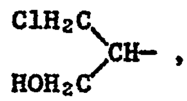 , 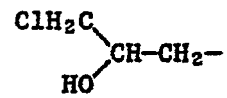

oder deren Mischungen bedeuten.

$R_f$ bedeutet bevorzugt lineares $C_6$–$C_{12}$-Perfluoralkyl, wobei auch Mischungen solcher Perfluoralkylgruppen in Frage kommen. Bevorzugt steht m für 2.

Die erfindungsgemässen Ester können nach bekannten Verfahren hergestellt werden. Man kann zum Beispiel 2 Mol eines Alkohols der Formeln IIIa oder IIIb

$R_fCH_2CH_2SCH_2CH_2CH_2OH$ (IIIa)
$R_fCH_2CH_2SO_2CH_2CH_2CH_2OH$ (IIIb)

mit Pyromellitsäuredianhydrid und 2 Mol Epichlorhydrin verestern. Das Verfahren kann in 2 Stufen ausgeführt werden.

Die Verbindungen der Formeln I und II können z.B. hergestellt werden, indem man die Alkohole der Formeln IIIa und/oder IIIb

$R_fCH_2CH_2SCH_2CH_2CH_2OH$ (IIIa)
$R_fCH_2CH_2SO_2CH_2CH_2CH_2OH$ (IIIb),

worin $R_f$ die zuvor definierte Bedeutung hat, mit Pyromellitsäuredianhydrid (PMDA) umsetzt, und die erhaltenen Diester der Formeln IV oder V

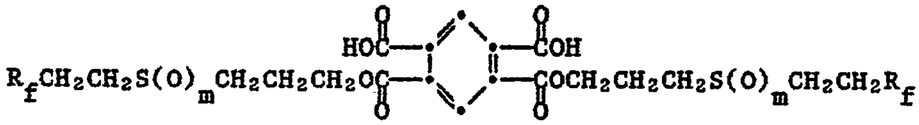 (IV)

oder

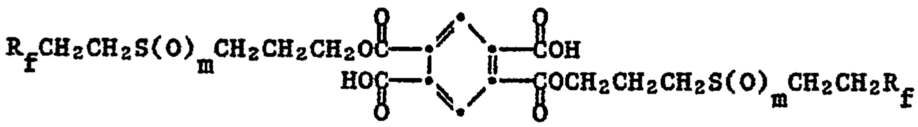 (V)

oder Mischungen davon danach mit Epichlorhydrin umsetzt.

In einer bevorzugten Ausführungsform werden die Diester der Formeln IV und V durch die Reaktion von 2 Mol Alkohol der Formeln IIIa oder IIIb mit 2 Mol PMDA hergestellt. Zwei Mol der Diester der Formeln IV und/oder V werden dann mit 2 Mol Epichlorhydrin umgesetzt, um die Ester der Formeln I und II zu erhalten. Die Epoxidgruppe des Epichlorhydrins kann an beiden C-Atomen substituiert werden. Das Reaktionsprodukt enthält daher im allgemeinen geringere Mengen eines Esters mit $HOCH_2CH(CH_2Cl)O(O)C$-Gruppen und grössere Mengen eines Esters mit $HOCH(CH_2Cl)CH_2(O)C$-Gruppen.

Bei den Diestern der Formeln IV und V handelt es sich um Meta- und Paraisomere. Dieses Diesterzwischenprodukt ist tatsächlich eine Mischung der Meta- und Paraisomeren. Es ist nicht notwendig, die Diestèr vor dem nächsten Verfahrensschritt zu isolieren. Falls gewünscht, können die Diester durch fraktionierte Kristallisation isoliert und das Isomerengemisch danach mittels bekannter Verfahren getrennt werden.

Sowohl die Reaktion der Alkohole der Formeln IIIa oder IIIb mit Pyromellitsäuredianhydrid, als auch die Umsetzung der dabei gebildeten Diester mit Epichlorhydrin werden vorteilhaft in Gegenwart eines inerten organischen Lösungsmittels durchgeführt. Geeignete Lösungsmittel sind zum Beispiel Dimethylformamid, N-Methylpyrrolidon, aliphatische Ester mit einem Siedepunkt unter 150°C, wie z.B. Essigsäuremethyl-, -ethyl- oder -propylester, und aliphatische Ketone wie z.B: Methylisobutylketon. Bevorzugt wird N-Methylpyrrolidon als Lösungsmittel verwendet.

Die Reaktion der Alkohole der Formeln IIIa oder IIIb mit Pyromellitsäureanhydrid wird vorteilhaft bei einer Temperatur von 20 bis 80°C, bevorzugt 40 bis 70°C durchgeführt. Die Reaktionszeit kann von der Reaktionstemperatur abhängen und im allgemeinen von 2 bis 40 Stunden betragen. Zur Reaktionsbeschleunigung können übliche basische Katalysatoren, z.B. tertiäre Amine mitverwendet werden. Beispiele hierfür sind Pyridin und Tri($C_1$–$C_6$-Alkyl)amine, besonders Triethylamin.

Die Reaktion der Diester der Formeln IV und V mit Epichlorhydrin wird vorteilhaft bei einer Temperatur von 20 bis 90°C, besonders 40 bis 60°C durchgeführt. Die Reaktionszeit kann etwa von 2 bis 20 Stunden betragen. Die Reaktion wird bevorzugt in Gegenwart eines basischen Katalysators durchgeführt, z.B. tertiären Aminen. Beispiele für solche Amine sind Pyridin oder Tri($C_1$–$C_6$-Alkyl)amine, z.B. Tributylamin und besonders Triethylamin.

Die beiden Reaktionsstufen können aufeinanderfolgend im gleichen Reaktionsgefäss durchgeführt werden. Vorteilhaft wird die Reaktion bei Normaldruck und unter trockenem Schutzgas durchgeführt, z.B. unter trockenem Stickstoff. Die Lösungsmittelmenge wird bevorzugt so bemessen, dass mindestens die Hälfte der Reaktanden und Zwischenprodukte in Lösung sind. Vorteilhaft wird soviel Lösungsmittel verwendet, dass sich alle Reaktanden, Zwischen- und Endprodukte in Lösung befinden.

Die Ester der Formel I und II können nach üblichen Verfahren isoliert werden. Durch Zugabe eines Nichtlösungsmittels können die Ester ausgefällt und flüchtige Lösungsmittel können abdestilliert werden. Wenn N-Methylpyrrolidon verwendet wird, kann der Ester durch Zugabe von Wasser ausgefällt und isoliert werden. Das erhaltene Produkt kann gereinigt werden, z.B. durch Waschen mit Wasser, um Verunreinigungen zu entfernen, die aus Lösungsmittel, Katalysator und/oder Ausgangs- und Zwischenprodukten bestehen können.

Die Ester der Formel I und II, in organischen Lösungsmitteln wie z.B. Aceton, Methanol oder Dioxan gelöst, oder in Form einer wässrigen Emulsion (vergleiche US-PS 4 283 292) können als Beschichtungsmittel für Polyamid- und Polyesterfasern verwendet werden. Sie können alleine oder zusammen mit anderen Faserbehandlungsmitteln verwendet werden. Ein Beispiel für solche Mittel sind Spinnappreturen, die ein Brechen der Faser bei Behandlungsverfahren vermindern.

Geeignete Polyamidfasern sind z.B. Fasern aus Poly(caproamid) (Nylon 6), Poly(hexamethylendiaminadipat) (Nylon 6,6) und andere Polyamide vom Typ der Poly(aminosäuren) und Poly(diamindicarboxylate), z.B. Poly(hexamethylendiaminsebacat) (Nylon 6,12). Geeignete Polyester sind z.B. Poly(ethylenterephthalate). Die Auftragsmenge an Estern der Formeln I und II beträgt bevorzugt 0,075–0,25% Fluor, bezogen auf das Gewicht der Fasern.

Nach der Beschichtung der Fasern werden diese zweckmässig getempert, um die Bindung der Schicht an die Fasern zu verbessern. Das Tempern wird im allgemeinen bei Temperaturen von 80 bis 160°C, vorzugsweise 100 bis 140°C vorgenommen.

Die thermische Stabilität der Ester der Formeln I und II, besonders jenen, die Sulfongruppen enthalten, ist besser als von Pyromellitsäureestern, die Perfluoralkylethangruppen enthalten. Der Kohlerückstand ist höher, was die verbesserte thermische Stabilität und eine verbesserte Flammwidrigkeit anzeigt.

Die erfindungsgemässen Ester verleihen synthetischen Polyamid- und Polyesterfasern einen ausgezeichneten Widerstand gegen Verschmutzung, die über zahlreiche Waschzyklen erhalten bleibt. Diese Widerstandsfähigkeit gegen Verschmutzung bleibt daher beim Gebrauch für einen längeren Zeitraum erhalten.

Ein weiterer Gegenstand der Erfindung ist ein Verfahren zur ölabweisenden und Schmutz widerstehenden Ausrüstung von synthetischen Polyamid- und Polyesterfasern, bei dem die Fasern mit einer wirksamen Menge einer Verbindung der Formeln I oder II oder Mischungen hiervon in Berührung gebracht werden.

Die nachfolgenden Beispiele erläutern die Erfindung näher. Alle Teile sind Gewichtsteile, sofern es nicht anders angegeben ist.

Beispiel 1: Pyromellitsäuredianhydrid (2,2 g; 0,01 Mol), ein Fluoralkohol der Formel $C_8F_{17}(CH_2)_2SO_2(CH_2)_3OH$ (11,4 g; 0,02 Mol), Dimethylaminopyridin (0,02 g) und Essigsäurebutylester (50 g) werden gemischt und unter $N_2$-Atmosphäre 24 Stunden bei 60°C gerührt. Danach wird das Lösungsmittel unter vermindertem Druck entfernt. Man erhält 12,4 g (91% Ausbeute) einer Mischung mit gleichen Anteilen an

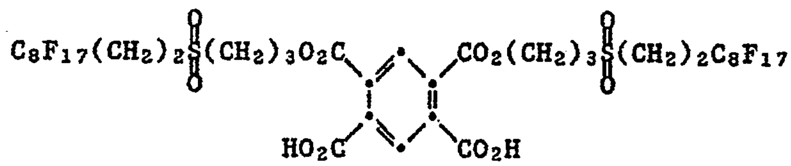

und

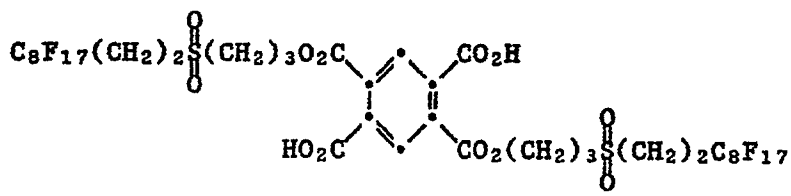

| $^1$H-NMR-Spektrum: | | |
|---|---|---|
| 2,02 ppm | Breiter Komplex | $O=C-O-CH_2CH_2-CH_2-SO_2$ |
| 2,77 ppm | Breiter Komplex | $C_8F_{17}-CH_2$ |
| 3,32 ppm | Komplex | $CH_2-SO_2$ |
| 4,41 ppm | Triplett | $O-COCH_2$ |
| 7,94 ppm | Komplex | Aromatische Protonen. |

Beispiel 2: Das gemäss Beispiel 1 erhaltene Produkt (21,4 g; 0,016 Mol) N-Methylpyrrolidon (150 g), Epichlorhydrin (8,7 g; 0,095 Mol) und Triethylamin (0,02 g) werden gemischt und während 6 Stunden bei 60°C umgesetzt.

Danach wird die dunkle Lösung in Wasser gegossen und der gebildete feste Niederschlag abfiltriert. Man erhält in 93% Ausbeute eine äquimolare Mischung von

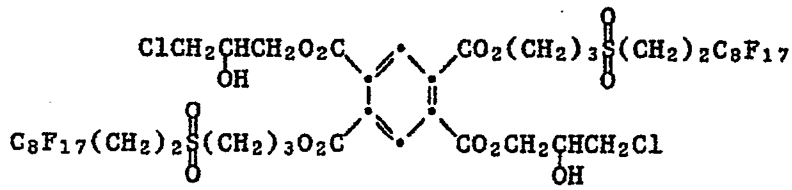

und

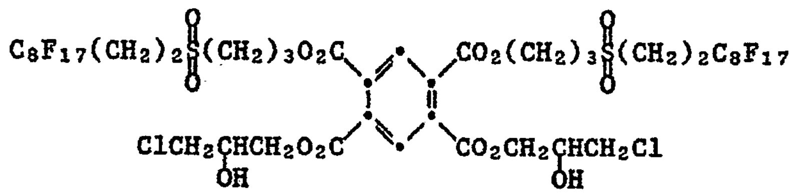

| $^1$H-NMR-Spektrum: | | |
|---|---|---|
| 1,92 & 2,19 ppm | Multipletts | $O-CH_2-\underline{CH_2}-CH_2S(O_2)$ |
| 2,69 ppm | Komplex | $C_8F_{17}-\underline{CH_2}-$ |
| 3,41–3,64 ppm | Komplex | $C_8F_{17}-CH_2-CH_2-S(O_2)-CH_2-$ |
| 4,34–4,41 ppm | Komplex | $O{=}C-O-\underline{CH_2}-$ |
| 7,82–8,61 ppm | Komplex | Aromatische Protonen. |

<u>Beispiel 3:</u> Pyromellitsäuredianhydrid (2,2 g; 0,01 Mol), ein Alkohol der Formel $C_8F_{17}(CH_2)_2S(CH_2)_3OH$ (10,2 g; 0,02 Mol), N-Methylpyrrolidon (120 g), Epichlorhydrin (7,2 g; 0,08 Mol) und Triethylamin (0,04 g) werden unter Stickstoff gemischt und die Mischung wird während 72 Stunden bei 60°C gerührt. Die erhaltene dunkle Lösung wird in 800 ml Wasser gegossen. Es bildet sich ein beiger Niederschlag, der abfiltriert wird. Der feste Niederschlag ist eine äquimolare Mischung aus

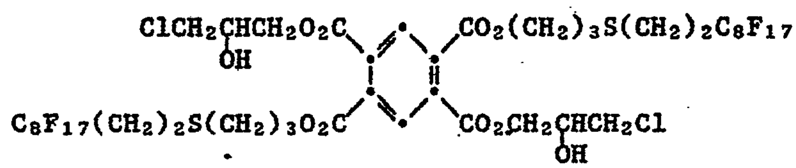

und

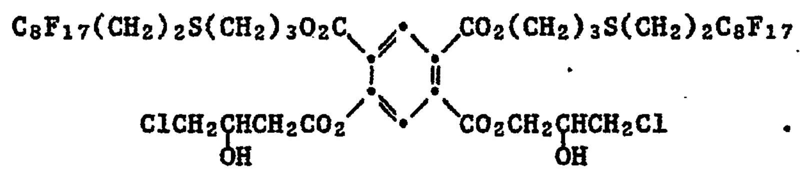

| $^1$H-NMR-Spektrum: | | |
|---|---|---|
| 2,05 ppm | Multiplett | $O-CH_2-\underline{CH_2}-CH_2-S-$ |
| 2,37 ppm | Komplex | $C_8F_{17}-\underline{CH_2}-$ |
| 2,71 ppm | Komplex | $C_8F_{17}-CH_2-\underline{CH_2}-S-\underline{CH_2}-$ |
| 4,16 ppm | Quintett | $-C(H)(OH)-$ |
| 4,47 ppm | Komplex | $O{=}C-O-\underline{CH_2}-$ |
| 8,07 ppn | Komplex | Aromatische Protonen. |

Beispiel 4: Die erfindungsgemässen Verbindungen werden für die Textil- und Teppichbehandlung verwendet. Es ist daher wünschenswert, dass sie eine verminderte Entflammbarkeit besitzen. Der Rückstand, der nach einer thermischen Zersetzung zurückbleibt (Kohlerückstand, char yield), ist ein Mass für die Entflammbarkeit. Der Kohlerückstand wird durch thermogravimetrische Analyse bestimmt (10°C/min., 10 ml $N_2$/min., 80% Unterdrückung). Das Ergebnis ist nachfolgend angegeben.

| Verbindung gemäss Beispiel | Kohlerückstand bei 400°C (% des ursprünglichen Gewichts) |
|---|---|
| 2 | 12 |

Beispiel 5: Die Verbindung gemäss Beispiel 3 wird als Veredelungsmittel für Teppiche in einer Reihe von Gehtesten auf Teppichen geprüft. Eine Lösung der Verbindung in Methylethylketon wird hierbei auf einen weissen büscheligen Nylonteppich aufgesprüht (0,175% F, bezogen auf das Gewicht des Teppichs). Das Ergebnis ist in Tabelle 1 angegeben.

Tabelle 1

| Probe | Geh-Test* | | | | Flüssigkeitsabweisung** | |
|---|---|---|---|---|---|---|
| | 1. Woche | 2. Woche | 3. Woche | 4. Woche | 10% Isopropanol in Wasser | Olivenöl |
| Kontrolle | 2–3 | 2 | 2 | 2 | – | – |
| Beispiel 3 | 4 | 3–4 | 3–4 | 3–4 | + | + |
| Anzahl der Schritte | 1910 | 3366 | 4466 | 7036 | | |

* Visuelle Beurteilung
1 = ungenügend, zieht eine Menge Schmutz an (schlechter als unbehandelte Probe)
2 = mässig
3 = gut
4 = sehr gut
5 = ausgezeichnet (praktisch wie ursprüngliche Probe).
** Widerstand des behandelten Teppichs gegenüber Flüssigkeiten, Tropfen werden aufgebracht.
+ = Bildung von Perlen
– = aufgesaugt

## Patentansprüche

1. Verbindungen der Formeln I oder II

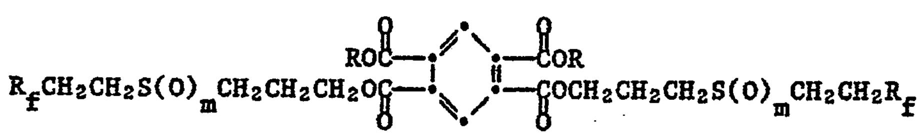 (I)

oder

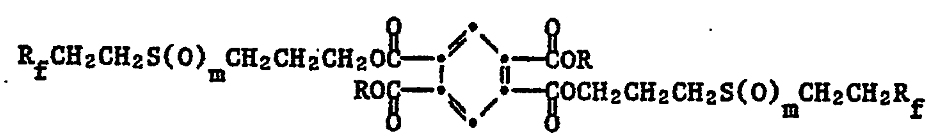 (II),

oder Mischungen davon,
worin
$R_f$ $C_3$–$C_{18}$-Perfluoralkyl, m 0 oder 2, und

R 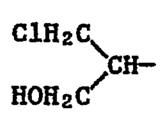 , 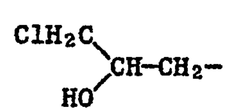

oder deren Mischungen bedeuten.

2. Verbindungen gemäss Anspruch 1, worin $R_f$ lineares $C_6$–$C_{12}$-Perfluoralkyl oder Mischungen davon bedeutet.

3. Verbindungen gemäss Anspruch 1, worin m 2 ist.

4. Verfahren zur Herstellung von Verbindungen der Formel I oder II gemäss Anspruch 1, bei dem man 2 Mol Alkohole der Formeln IIIa oder IIIb

$R_fCH_2CH_2SCH_2CH_2CH_2OH$ (IIIa)
$R_fCH_2CH_2SO_2CH_2CH_2CH_2OH$ (IIIb)

mit 1 Mol Pyromellitsäuredianhydrid und 2 Mol Epichlorhydrin umsetzt.

5. Verfahren zur ölabweisenden und Schmutz widerstehenden Ausrüstung von synthetischen Polyamid- und Polyesterfasern, bei dem die Fasern mit einer wirksamen Menge einer Verbindung der Formeln I oder II oder Mischungen hiervon gemäss Anspruch 1 in Berührung gebracht wird.

## Claims

1. A compound of the formulae I or II

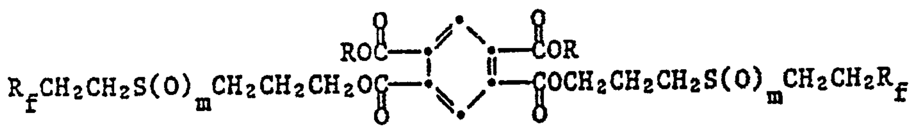

(I)

or

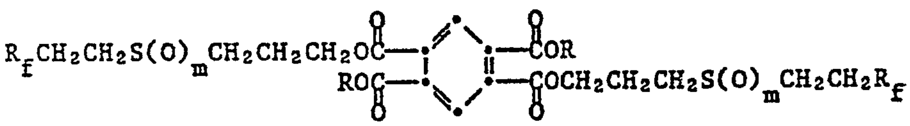

(II),

or a mixture thereof, wherein $R_f$ is $C_3$–$C_{18}$-perfluoroalkyl, m is 0 or 2 and

R is 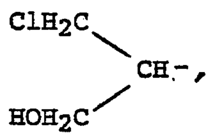, 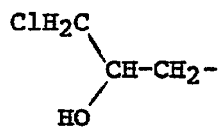

or mixtures thereof.

2. A compound according to claim 1, wherein $R_f$ is linear $C_6$–$C_{12}$-perfluoroalkyl, or a mixture thereof.

3. A compound according to claim 1, wherein m is 2.

4. A process for the preparation of a compound of the formula I or II according to claim 1, which comprises reacting 2 moles of alcohols of formulae IIIa or IIIb

$R_fCH_2CH_2SCH_2CH_2CH_2OH$ (IIIa)
$R_fCH_2CH_2SO_2CH_2CH_2CH_2OH$ (IIIb)

with 1 mole of pyromellitic dianhydride and 2 moles of epichlorohydrin.

5. A method of oil-repellant and soil-resistant finishing of synthetic polyamide or polyester fibres, by bringing the fibres into contact with an effective amount of a compound of the formulae I or II or mixtures thereof according to claim 1.

## Revendications

1. Composés qui répondent à l'une ou à l'autre des formules I et II:

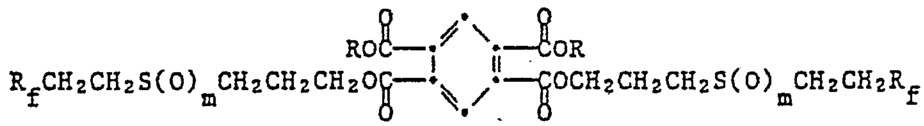

(I)

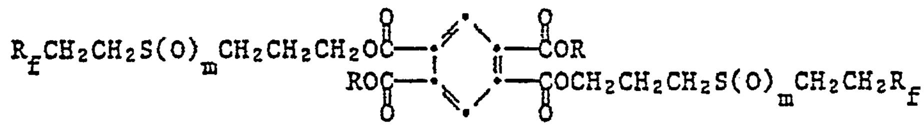

(II)

et mélanges de tels composés, formules dans lesquelles:
$R_f$ représente un radical perfluoroalkyle en $C_3$–$C_{18}$,
m est égal à 0 à 2, et

R représente 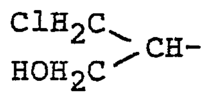 , 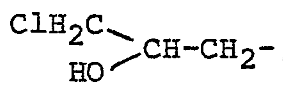

ou leurs mélanges.

2. Composés selon la revendication 1 dans lesquels $R_f$ représente un radical perfluoroalkyle linéaire en $C_6$–$C_{12}$ ou des mélanges de tels radicaux.

3. Composés selon la revendication 1 dans lesquels m est égal à 2.

4. Procédé pour préparer des composés de formules I ou II selon la revendication 1, procédé selon lequel on fait réagir 2 mol d'alcools de formules IIIa ou IIIb:

$R_fCH_2CH_2SCH_2CH_2CH_2OH$ (IIIa)
$R_fCH_2CH_2SO_2CH_2CH_2CH_2OH$ (IIIb)

avec 1 mol de dianhydride pyromellitique et 2 mol d'épichlorhydrine.

5. Procédé pour conférer un ennoblissement oléofuge et antisalissure à des fibres de polyesters et de polyamides synthétiques, procédé selon lequel on met les fibres en contact avec une quantité efficace d'un composé de formule I ou II ou de mélanges de ceux-ci selon la revendication 1.